# EUROPEAN PATENT APPLICATION

(11) **EP 4 480 520 A1**
(43) Date of publication of application: **25.12.2024**
(21) Application number: 23461607.6
(22) Date of filing: 18.06.2023
(51) Int. Cl.: A61M 15/00, A61M 16/00, A61M 16/08, A61M 16/06

(54) **MONITORING SYSTEM FOR ASSISTING AND MONITORING THE NEBULISATION PROCESS**

(71) Applicant: Uniwersytet Zielonogórski, 65-417 Zielona Góra (PL)
(72) Inventor: Doligalski, Michal, 65-395 Zielona Gora (PL); Mrugalski, Marcin, 65-128 Zielona Gora (PL); Lukasik, Marcin, 66-016 Czerwiensk (PL)
(74) Representative: JD&P Patent Attorneys Joanna Dargiewicz & Partners

(57) **Abstract**

The object of the present invention is a monitoring system for assisting and monitoring the nebulisation process comprising a pass-through adapter with integrated sensors, a pressure sensor, a temperature sensor, a microprocessor system built from a pressure analysis block, a temperature analysis block, a decision block, an interface circuit, a display, data lines (instantaneous pressure data line, instantaneous temperature data line, pressure feedback line, temperature feedback line, pressure data line, temperature data line, system data line, display data line, external data line), characterised in that a pass-through adapter is installed between the outlet port of the nebulisation vessel and an element for delivering the nebulisation solution to the patient, such as a mask, a nasal adapter, a circular tube; the pressure sensor located in the adapter collects the pressure information at the inlet port and transmits the collected data via the line to the pressure analysis block, the pressure analysis block is triggered and controlled via a feedback loop from the decision block, and determines the data in the form of minimum pressure, maximum pressure and the patient's inhalation and exhalation frequency, the temperature analysis block is triggered and controlled by a feedback loop from the decision-making block and determines data in the form of minimum, maximum and instantaneous temperature values, which are passed on via the line to the decision-making block; the decision block determines from the PD and TD data whether the nebulisation process is proceeding correctly, relates this data to the temperature data and transmits it to the interface circuit via the system data line and determines a recommendation for the patient in the form of information such as: increase expiratory rate, decrease expiratory rate, increase inspiratory depth, decrease inspiratory depth and transmits it via the system line to the interface circuit.

## Description

The object of the present invention is a monitoring system for assisting and monitoring the nebulisation process.

Aerosol therapy involves the delivery of medication (active substance) by inhalation into the patient's airways in the form of a mist, with distilled water usually acting as the carrier. The therapy is usually carried out at home, without the supervision of medical staff, by unqualified persons. In particular, nebulisation therapy in children is problematic. For it to be effective, two conditions must be met: the patient should take deep breaths at a low frequency and the temperature of the mist should be close to the patient's body temperature (a low temperature can cause bronchospasm). Existing inhaler solutions do not provide the possibility to monitor the nebulisation process. In order to fully control the nebulisation process:
1. it is necessary to monitor the process in terms of frequency and depth of inspiration;
2. it is necessary to monitor the temperature of the mist delivered to the patient's airways;
3. it is necessary to monitor the nebulisation process and the effectiveness of aerosol therapy by healthcare personnel using e-health systems;
4. it is necessary to support the nebulisation process by providing the patient with information on the correctness of the process and guidance on what changes should be made to make the process more effective (depth and frequency of inhalations).

### Prior art

International patent application WO9930760A1 discloses a device and method for providing a facemask fit to a patient's face, wherein the device comprises: a facemask having an inlet through which gas inhaled by the patient can be supplied; and a sensor for measuring the flow rate of gas drawn through the inlet of the facemask; wherein the fit of the facemask is determined by monitoring the flow rate of gas drawn through the inlet of the facemask during inhalation by the patient, the facemask being considered satisfactory in terms of fit to the patient when a substantially regular inhalation waveform is achieved. The device according to the invention tests the fit of the patient's mask by checking the gas flow during the inhalation and exhalation phases. The device primarily uses a gas flow sensor. The purpose of the device is to check that the mask is fitted and that there are no leaks through lack of fit.

Canadian patent application CA3166438A1 describes methods and apparatus for measuring a breath sensor. One embodiment may generally include a sampling unit having a breath sampling port, at least one pressure sensor disposed in the sampling unit and coupled to the breath sampling port, and a processor coupled to the at least one pressure sensor. The processor may be configured to prompt the user with instructions to exhale the breath sample for a first predetermined period of time into the breath sampling port and to inhale for a second predetermined period of time through the breath sampling port. The processor may be configured to measure the change in pressure using the pressure sensor during the first and second predetermined time periods such that the processor determines a total volume of air corresponding to the lung capacity of the user based on the change in pressure during the first and second predetermined time periods. The device is used to measure biological parameters through breath testing. In particular, to measure the carbon monoxide (presence and level) contained in the exhaled air. The device is intended, for example, to identify the smoking behaviour of tobacco (cigarette) smokers. In addition, the device, by measuring the volume of inhaled air, can be used to conduct spirometry, which is used to assess the conditions of patients with asthma, pulmonary fibrosis, cystic fibrosis, COPD. The solution is used to measure the typical data associated with spirometry (FEVI/FVC), and is used to diagnose conditions (including severity) rather than support at the nebulisation (aerosol therapy) stage of the process.

International patent application WO2021022785A1 discloses a multifunctional oxygen delivery system based on a common respiratory rate and blood oxygen target, consisting mainly of a control module, a multi-parameter monitoring module, an electronic flow valve, an airflow diversion switch, a human-computer interaction interface and a communication module. The device is used to increase the efficiency of oxygen delivery in oxygen therapy by correlating the opening of oxygen delivery valves. The device tests the effect of oxygen therapy by measuring the oxygen concentration in the blood (oxygen saturation).

International patent application WO0176762A2 discloses a nebuliser for spraying liquid solutions or suspensions. The device uses electronic circuitry to control the aerosol generation efficiency of the ultrasonic nebuliser by testing electrical parameters.

Polish patent application P395401 discloses a method and a device for controlling and monitoring an aerosol generation device for administering a dose of a substance by inhalation. The invention relates to a method for controlling and monitoring an aerosol generation device for administering a dose of a drug by inhalation by means of an aerosol generation control and dose monitoring device, wherein any type of supervised aerosol generation device for administering a dose of a drug by inhalation is used, preferably a metered dose nebulizer, an ultrasonic inhaler or any other inhaler of a similar type with known performance characteristics, whereby during aerosol generation at metered dose by means of the supervised device, the exceedance of a threshold dose of pressure in the pressure tubing is read, connected to the mouthpiece of the supervised device, which signifies the start of the inhalation phase and triggers aerosol generation by the supervised inhalation device until the threshold pressure in the pressure tube is again exceeded, which is read as an exhalation phase or respiratory pause, characterised in that the supervised device is combined in an assembly with a device for controlling aerosol generation and monitoring the dosage dispensed, which assembly is controlled according to the respiratory phases of inhalation and exhalation. The device uses pressure measurement to determine aerosol delivery and also to measure the drug administered. However, the device does not allow the efficacy of aerosol therapy to be influenced by monitoring the process (inhalation frequency, depth, temperature measurement) and providing guidance to the patient. It also does not allow analysis of the effectiveness of the therapy by the doctor.

As can be seen, solutions are known in the state of the art indicating a correlation between the frequency and depth of the patient's inhalations and the aerosol temperature during aerosol therapy and the effect on the efficacy of the therapy, but there is no disclosure of a method of using data about the nebulisation process (frequency of inhalations, depth of inhalations, aerosol temperature) to monitor in real time the nebulisation process and translate this into indications given to the patient to increase the efficacy of the therapy and also to use the collected data to evaluate the efficacy of the therapy by the physician as in the object of the invention. None of the state of the art documents disclose a method for monitoring the nebulisation process and assisting the process by analysing parameters such as inhalation frequency and depth in combination with monitoring the aerosol temperature as in the object of the invention.

There are currently no ways for assisting and monitoring the nebulisation process by analysing in real time parameters such as inspiratory frequency and depth, as well as the aerosol temperature value, and giving real-time indications to the patient.

So, the aim of the present invention was to develop a monitoring system for assisting and monitoring the nebulisation process, which will increase the efficiency of the nebulisation process.

This objective is achieved by the object of the present invention, which is a monitoring system for assisting and monitoring the nebulisation process comprising a pass-through adapter with integrated sensors, a pressure sensor, a temperature sensor, a microprocessor system built from a pressure analysis block, a temperature analysis block, a decision block, an user interface circuit, a display, data lines (instantaneous pressure data line, instantaneous temperature data line, pressure feedback line, temperature feedback line, pressure data line, temperature data line, system data line, display data line, external data line), characterised in that the through-hole adapter is installed between the outlet port of the nebulisation vessel and an element for delivering the nebulisation solution to the patient, such as a mask, a nasal adapter, a circular tube; the pressure sensor located in the adapter collects information about the pressure in the inlet port and transmits the collected data via the line to the pressure analysis block, the pressure analysis block is triggered and controlled via a feedback loop from the decision block, and determines data in the form of minimum pressure, maximum pressure and the patient's inhalation and exhalation frequency, The temperature analysis block is triggered and controlled by a feedback loop from the decision-making block and determines data in the form of minimum, maximum and instantaneous temperature values, which are passed on to the decision-making block via the line; the decision block determines from the PD and TD data whether the nebulisation process is proceeding correctly, relates this data to the temperature data and transmits it to the user interface circuit via the system data line and determines a recommendation for the patient in the form of information such as: increase expiratory rate, decrease expiratory rate, increase inspiratory depth, decrease inspiratory depth and transmits it via the system line to the interface circuit.

Examples of the realisation of a monitoring system for assisting and monitoring the nebulisation process according to the present invention are shown in the figures of the figure, where:
Fig. 1 shows a circuit with a touchscreen display, a
Fig. 2 shows a circuit with a non-touchscreen display equipped with an IDM interface.

In one realisation example, the monitoring system for assisting and monitoring the nebulisation process according to the present invention comprises the following components:
- AD - through adapter with integrated sensors
   ∘ P - pressure sensor
   ∘ T - temperature sensor
- MS - microprocessor-based system comprising:
   ∘ PA - pressure analysis block
   ∘ TA - temperature analysis block
   ∘ BD - decision-making block
   ∘ Ul - user interface
- TSD - display, in particular touchscreen display
- data lines:
   ∘ PL - instantaneous pressure data line
   ∘ TL - instantaneous temperature data line
   ∘ LP - pressure feedback line
   ∘ LT - temperature feedback line
   ∘ PD - Pressure data line
   ∘ TD - Temperature data line
   ∘ SD - System data line
   ∘ DD - Display Data line
   ∘ ED - external data line (ED).

The through adapter (AD) is placed between the outlet port of the nebulisation vessel and the element delivering the nebulisation solution to the patient (mask, nasal adapter, round tube). The pressure sensor (P) located in the Adapter (AD) collects information about the pressure in the inlet port and the collected data is transmitted via the line (PL) to the pressure analysis block (PA). The pressure analysis block (PA) is triggered and controlled via a feedback loop (LP) from the decision block. The pressure analysis block (PA) determines data in the form of minimum pressure, maximum pressure (amplitude) and the patient's inspiratory and expiratory frequency. This information, via the line (PD), is passed to the decision block.

The temperature analysis block (TA) is triggered and controlled by a feedback loop (LT) from the decision block. The temperature analysis block (PA) determines data in the form of minimum, maximum and instantaneous temperature values. This information is transmitted via the line (TD) to the decision block.

The decision block (BD) determines from the PD and TD data whether the nebulisation process is proceeding correctly, i.e. whether the inhalation and exhalation rates are correct and whether the amplitude of the inhalations and exhalations is correct. The decision block relates this data to the temperature data and transmits it to the interface circuit (Ul) via the system data (SD) line. The decision block also determines a recommendation for the patient in the form of information such as: increase expiratory rate, decrease expiratory rate, increase inspiratory depth, decrease inspiratory depth and transmits it via the system data (SD) line to the interface circuit.

The decision block (BD), by means of the LP and LT feedback loops, controls the PA and TA blocks, in particular the frequency of calculation of the pressure and temperature parameters and triggers readings - synchronising the temperature and pressure values, while being able to take temperature and pressure data synchronously and independently.

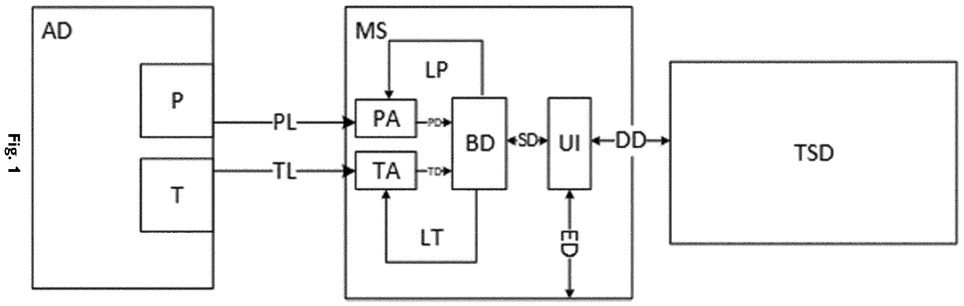

The interface circuit (Ul) is equipped with a memory, storing data on the current nebulisation process. The aim of the user interface circuit is to prepare actual data from the nebulisation process and recommendations for the patient and transmit them via the display data line (DD) to the display, in particular the touch display. The touchscreen display (TSD) presents the data to the patient to support the nebulisation process (Fig. 1).

The user interface circuit (Ul), via the external data (ED) line, transmits data about the nebulisation process to other subsystems (enables integration) in particular to systems that enable data storage and analysis in the cloud.

The user interface (Ul) system receives data from the touchscreen display via the DD line, including set limit parameters of temperature, pressure, frequency. The exchange of data via the external data (ED) line enables the invention to be used as a larger component of an Internet of Things (IoT) system that is part of an e-health system.

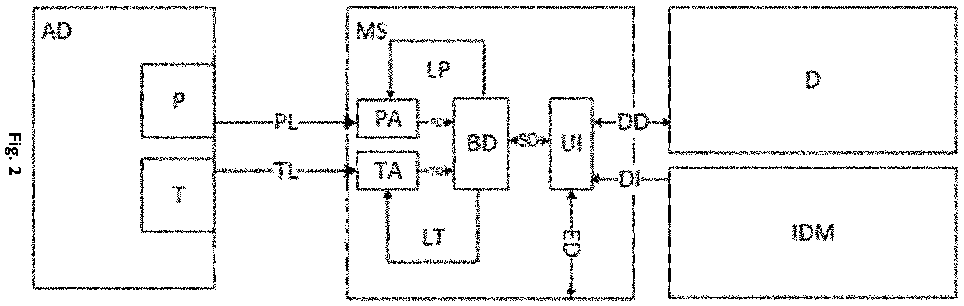

In another realisation example, the TSD touch display can be replaced by a display without touch functionality (D) (Fig. 2). In this case, a variant of the invention is equipped with the display D, an input data interface (IDM) (in particular pushbuttons, keypad, voice control interface) through which set parameters (temperature, pressure, frequency) are output.

In the present invention, an analysis of the frequency and depth of inhalation of the patient is performed, which, in combination with the measurement of the aerosol temperature, is intended to determine the correctness of the patient's inhalation, i.e. the depth of inhalation and its frequency.

Furthermore, the combination of pressure data together with aerosol temperature data, which influences the diameter of the aerosol droplets and thus the effectiveness and location of deposition in the patient's airway, is unique. The combination with aerosol temperature monitoring allows both the correction of breathing by the patient during the aerosol therapy process and the analysis of the measurement data by the physician to determine the effectiveness of the therapy. In the present invention, the acquisition of information about the correctness of the nebulisation process and its correction (including correction during the process itself) is crucial.

The monitoring system for assisting and monitoring the nebulisation process according to the present invention has two main functions:
1. It monitors the nebulisation process and communicates the monitoring results to the patient via a display and to the doctor via an e-health system.
2. Provides guidance to the patient on the depth of inhalation and its frequency.

## Claims

1. A monitoring system for assisting and monitoring the nebulisation process comprising:
• a through adapter (AD) with integrated sensors
∘ Pressure sensor (P)
∘ Temperature sensor (T)
• a microprocessor system (MS) comprising:
∘ pressure analysis block (PA)
∘ temperature analysis block (TA)
∘ decision-making block (BD)
∘ the user interface (Ul)
• a display (TSD, D)
• data lines:
∘ instantaneous pressure data line (PL)
∘ instantaneous temperature data line (TL)
∘ pressure feedback line (LP)
∘ temperature feedback line (LT)
∘ pressure data line (PD)
∘ temperature data line (TD)
∘ system data line (SD)
∘ display data line (DD)
∘ external data line (ED),
**characterized in that**
- the through adapter (AD) is installed between the outlet port of the nebulisation vessel and a component for delivering the nebulisation solution to the patient, such as a mask, nasal adapter, round tube;
- the pressure sensor (P) located in the adapter (AD) collects information about the pressure in the inlet port and transmits the collected data via the line (PL) to the pressure analysis block (PA);
- the pressure analysis block (PA) is triggered and controlled via a feedback loop (LP) from the decision block, and determines data in the form of minimum, maximum pressure and the patient's inspiratory and expiratory frequency, which is passed on via the line (PD) to the decision block (BD);
- the temperature analysis block (TA) is triggered and controlled by a feedback loop (LT) from the decision block, and determines data in the form of minimum, maximum and instantaneous temperature values, which are transmitted via the line (TD) to the decision block;
- the decision block (BD) determines from the PD and TD data whether the nebulisation process is proceeding correctly, relates this data to the temperature data and transmits it to the interface circuit (Ul) via the system data (SD) line and determines a recommendation for the patient in the form of information such as: increase expiratory rate, decrease expiratory rate, increase inspiratory depth, decrease inspiratory depth and transmits it via the system data (SD) line to the interface circuit.

2. The system according to claim 1, **characterised in that** the display is a touch screen display (TSD).

3. The system according to claim 1, **characterised in that** the display is a non-touch display (D) and the system additionally contains an input data interface (IDM), in particular buttons, keyboard, voice control interface, through which set parameters (temperature, pressure, frequency) are output.
